# EUROPEAN PATENT APPLICATION

(11) **EP 1 291 656 A2**
(43) Date of publication of application: **12.03.2003**
(21) Application number: 02256170.8
(22) Date of filing: 05.09.2002
(51) Int. Cl.: G01N 33/543, G01N 33/551, G01N 33/548, C12Q 1/68

(54) **Biomolecule array**

(30) Priority: 06.09.2001 GB 0121602
(71) Applicant: Randox Laboratories Ltd., County Antrim, BT29 4QY, Northern Ireland (GB)
(72) Inventor: Benchikh, Elouard, Randox Lab. Ltd., Co. Antrim BT29 4QY, Northern Ireland (GB); McConnell, Robert Ivan, Randox Lab. Ltd., Co. Antrim BT29 4QY, Northern Ireland (GB); Lamont, John Victor, Randox Lab. Ltd., Co. Antrim BT29 4QY, Northern Ireland (GB); Fitzgerald, Stephen Peter, Randox Lab. Ltd., Co. Antrim BT29 4QY, Northern Ireland (GB)
(74) Representative: Jappy, John William Graham

(57) **Abstract**

Multi-analyte microarrays are produced by first attaching to the surface of a solid support, a silane with an electrophilic terminal group, contacting the terminal group with a polyamine, and activating the polyamine with an electrophilic, bifunctional linker.

## Description

### Field of the Invention.

This invention relates to the preparation of biomolecule arrays, in particular to DNA arrays.

### Background to the Invention.

Diagnostic tests are recognised as increasingly important. Multi-analyte systems have been developed in order to move away from the traditional one-analyte, one-test methods of analysis. These multi-analyte systems should provide a method for high-throughput, efficient and effective analysis of multiple compounds to be detected. A multi-analyte device should consist of a solid substrate upon which numerous test regions are available for detection of various analytes. These analytes should be able to be detected from a single test sample.

It is now common practice to use a solid support for the immobilisation of a biomolecule of interest. Immobilisation offers many advantages, including a vast range of different surface chemistries that can be used to bind the biomolecule to the surface. One ofthe most common methodologies that has been employed to initialise chemical reactivity is that of silanation of solid supports to provide a reactive terminus. Most commonly, this reactive terminus is electrophilic in nature, allowing it to react with nucleophiles (usually amines) that are commonly found, or can be introduced into, the biomolecule of interest (see for example GB-A-2324866).

An alternative is to silanate the surface with an agent that provides a nucleophilic end-group (see, for example, WO-A-99/51773). Although such end-groups are inert towards most biomolecules, the surface can be activated with a bifunctional linker which possesses two electrophilic termini separated by a linking moiety. The biomolecule can then be bound to the surface.

Although in theory the silanating agent should provide an even monolayer over the substrate, in practice many competing side reactions mean that the surface may not be as homogeneous as desired. One option that has been pursued is to cover the initial silane surface with a reactive polymer to form an additional, covering layer. US 6150103 describes the use of polyethyleneimine to cover the initial surface. A drawback to this approach is that the spotting solutions have to contain large concentrations of thickening agent to allow spotting onto the hydrophilic surface. The arrays produced are far from satisfactory in terms of spot quality.

There is a general need for improvements to the production of biomolecule arrays, to provide more defined spotting, to improve the quality of the arrays.

### Summary of the Invention

The present invention uses a combination of silanation, polymeric coating and surface activation in order to provide a homogenous surface that not only enhances signal intensity (e.g. from an oligonucleotide hybridisation assay), but also produces a surface that is amenable to quality, reproducible micro-array production.

According to a first aspect of the invention, a process for the manufacture of a solid support that contains a linker molecule capable of attaching a biomolecule to the solid support, comprises:
attaching to the surface of a solid support, a silane with an electrophilic terminal group;
contacting the terminal group with a polyamine; and
activating the polyamine with an electrophilic, bifunctional linker.

The use ofthese components promotes the signal intensity enhancement of discrete test regions, and the choice of bifunctional linker helps to improve spot quality.

According to a second aspect of the invention, a functionalised solid support material is obtainable by carrying out the process defined above.

### Description of the Drawings

The description is illustrated with reference to the following drawings, wherein:
Figure 1 is a schematic representation of the silanation of a solid support surface with (a) glycidoxypropyltriethoxysilane (GOPTS) and (b) glycidoxypropylmethyldimethoxysilane (GOPS MDM);
Figure 2 is a schematic representation of a silanated surface modified with aminated polymers; and
Figure 3 is a schematic representation of the activation of an aminated polymeric surface with 1,4-phenylenediisothiocyanate.

### Description of the Invention

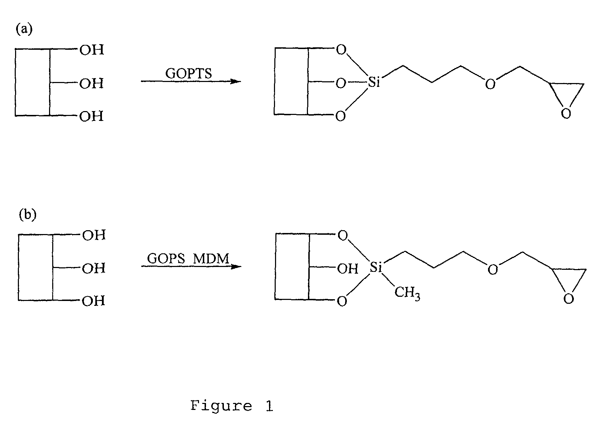

The approach of the invention uses a combination of silanation, polymeric coating and surface activation to produce a surface that can be tailored to provide a suitable solid substrate for, among other things, oligonucleotide hybridisation assays. The silanes useful in the invention comprise an electrophilic terminal group. Suitable silanes will be apparent to the skilled person. In a preferred embodiment, the silanes are alkoxy silanes terminated with electrophilic functionalities capable of reacting with polymeric amines. In a further preferred embodiment, the two main silanes used are glycidoxypropyltriethoxysilane (GOPTS) and glycidoxypropylmethyldimethoxysilane (GOPS MDM). These silanes contain alkoxy functionalities that couple directly with a solid ceramic substrate via a condensation reaction to form stable linkages. They are also terminated with an epoxide ring that is able to react irreversibly with amines (see Fig. 1).

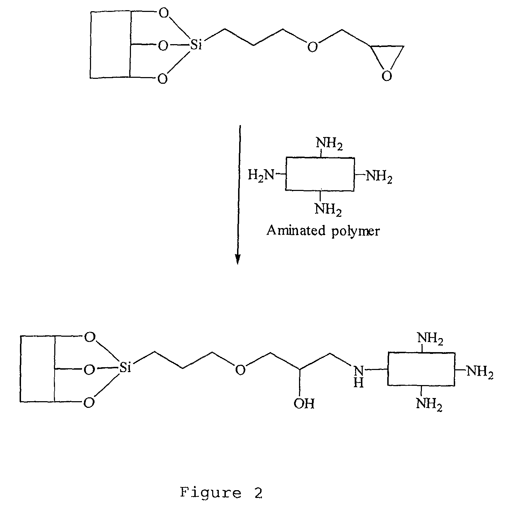

Any suitable polyamine may be used. Polyamines suitable for use in the invention will be apparent to the skilled person. An example of a suitable polyamine is polyethyleneimine (PEI, molecular weight ≈ 25000) which has an unknown structure. Amino-modified dextran (molecular weight 40000), with a linear, rigid structure is preferred. Non-linear molecules, for example, generations 3 and 4 of a spherical, amine-terminated dendrimer are also preferred. These polymers should affect the hybridisation of oligonucleotides in a varied manner (see Fig. 2).

The polymers formed by reaction of the silanes and polyamines are activated with an electrophilic, bifunctional linker in order to elicit binding with, for example, amino-capped oligonucleotides. Suitable electrophilic bifunctional linkers will be apparent to the skilled person.

Preferably, the bifunctional linker is of the general formula:

X-R-X

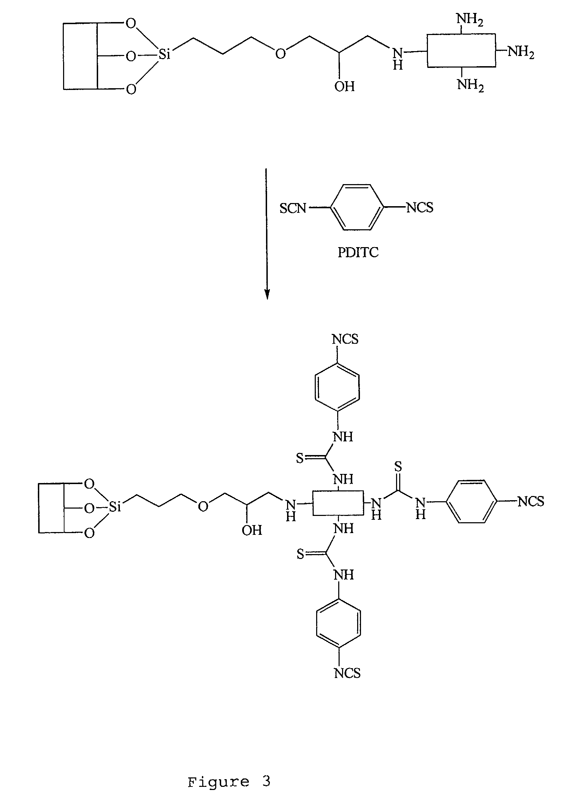

where R represents an alkyl group (e.g. C₁₋₆ alkyl), an aryl group (e.g. C₆H₄ phenyl) or a heteroaryl group, and X is selected from the group consisting of isothiocyanate, isocyanate, epoxide, carbonylimidazole and N-hydroxysuccinimide. The preferred embodiment, 1 ,4-phenylenediisothiocyanate (PDITC), is used to provide a means of rapid, irreversible immobilisation of the amino-capped biomolecules (oligonucleotides) (see Fig. 3).

The biomolecules that are to be attached to the solid support include proteins, peptides, antibodies and polynucleotides (oligonucleotides), e.g. DNA or RNA. Other suitable biomolecules will be apparent to the skilled person.

The invention is particularly suited for the incorporation of polynucleotides, which may be used in hybridisation assays to detect or characterise a target polynucleotide. The arrays of the invention give improved signal intensity when used in hybridisation assays.

Without wishing to be bound by theory, there appears to be three main possibilities why coverage of the silanated surface with amino-functionalised polymers may help to increase the signal attenuation from a hybridisation assay. The first proposal for the observed signal intensity increase is that the polymer coating will increase surface uniformity.

The second theory concerns the distance between the hybridisation event and the silanated surface. In particular, it has been reported that hybridisation between two complementary oligonucleotide strands is appreciably affected by steric hindrance and that for effective and efficient hybridisation to occur upon a solid surface, a spacer must be incorporated onto the surface to move the bound oligonucleotide away from steric interferences (Shchepinov *et al*., Nucleic Acids Res., 1997; 25:1155). It may be that the incorporation of an amino-functionalised polymer acts as an effective spacing moiety between the oligo and the solid surface and thus the efficiency of hybridisation is increased and signal intensities are enhanced.

The final explanation regarding the increase of signal intensities after polymer coating concerns the number of groups on the surface that are available for binding the capture (target) oligonucleotide. After silanation of the surface with an electrophilic silane, there are a finite number of groups on the surface that are available for coupling. However, after treatment with polymeric agents, the number of functionalities on the surface may increase, as the reaction of one electrophilic silane molecule with one polymeric amine group will still leave multiple amine functionalities uncoupled to the surface and available for activation. This higher surface functionality should increase the surface density of activated groups, raising the amount of nucleotide on the surface and hence should increase the relative signal intensity.

The following Example is provided for illustration only, and does not limit the scope of the invention.

### Example

### Silanation of the substrates

Pre-treated ceramic substrates were immersed in 4L of o-xylene containing 2% (v/v) 3-glycidoxytriethoxypropylsilane and 0.2% (v/v) of N-ethyldiisopropylamine and this was heated at 60°C for 5 hours before the solution was left to cool to room temperature overnight with the substrates still immersed. After removal of the silanation solution, the substrates were washed x1 toluene and x 2 acetone (10 minutes each wash) before being placed in a dessicator under vacuum for 24 hours.

### Modification via aminated polymer

The ceramic sheets were immersed in a 0.2% (w/v) solution of the appropriate aminated polymer in 50 mM carbonate solution (pH9.6) and this was allowed to react overnight at room temperature with shaking. The sheets were then washed with x 3 carbonate buffer and x 2 dd H₂O and then rinsed with acetone and allowed to dry at 37°C for 2 hours. The sheets were then ready for activation.

### Activation via bifunctional linkers

The amino modified sheets were immersed in a 1% (w/v) solution of the appropriate bifunctional linker in acetonitrile containing 1% (v/v) of N-ethyldiisopropylamine and this was reacted at room temperature for 6 hours before washing with 3 x acetonitrile and 2 x acetone. The sheets were dried overnight at 37°C prior to spotting.

### Oligonucleotide hybridisation assay conditions

The sheets were spotted with amino-functionalised capture oligo (50, 10 and 1µM in carbonate buffer, pH 9.6) before being dried for 2 hours at 37°C. The substrates were placed in a 5% ethanolamine solution, with shaking, for 30 minutes. They were washed with x 3 ddH₂O and x 2 5XSSC (5 minutes shaking each wash) and the sheets placed in a pre-heated 5XSSC solution containing 50 pmol/L of complementary digoxygenin-labelled oligo. This was incubated at 57°C and then washed with 2 x 5XSSC and 2 x ddH₂O (10 minutes each wash). At this point the chips were chopped and placed in their individual wells. 800 µL of 1% (w/v) casein in 1 x PBS was added with shaking for 30 minutes followed by 500 µL of the appropriate concentration of anti-digoxygenin-HRP (1/1k, 1/2k, 1/3k - incubated with shaking for 30 minutes at room temperature). The chips were washed with 3 x maleic acid buffer/0.03% Tween 20 and 2 x ddH₂O (5 minutes shaking each wash) and finally visualised after addition of chemiluminescent reagent, 15s exposure time on a CCD camera.

As well as spotting onto the activated polymeric surfaces, two control surfaces were also used for comparison. They were unmodified GOPTS and a nucleophilic silane, aminopropyltriethoxysilane (APTES), activated with PDITC. These were chosen to examine the performance of surfaces that did not have the additional polymeric coating step. The signal intensities are given in relative light units (rlu's).

**Table 1.**

| Signal intensities for different surfaces used in DNA hybridisation studies. | |
|---|---|
| **Surface** | **Signal Intensity (rlu)** |
| GOPTS | 29408 |
| APTES + PDITC | 17448 |
| GOPTS + PEI + PDITC | 22665 |
| GOPS MDM + PEI + PDITC | 16983 |
| GOPTS + Dextran 40k + PDITC | 39006 |
| GOPS MDM + Dextran 40k + PDITC | 58632 |
| GOPTS + Generation 3 dendrimer + PDITC | 38506 |
| GOPS MDM + Generation 3 dendrimer + PDITC | 44341 |
| GOPTS + Generation 4 dendrimer + PDITC | 59251 |
| GOPS MDM + Generation 4 dendrimer + PDITC | 58406 |

Close examination of the table of results shows that some important trends become apparent. Firstly, low signal intensities are found with the GOPTS and APTES/PDITC control surfaces. This is to be expected due to their lack of polymeric coating that is essential for reasons of increasing the spacer length between the surface and hybridisation and increasing the amount of functionality available on the surface for binding. More surprisingly, the signal intensities from those surfaces coated with polyethyleneimine prior to activation also show signal intensities that are in the same range as those observed with the two control surfaces. The polymeric PEI is expected to coat the surface evenly and provide a means for greater signal attenuation for the reasons discussed earlier. However, PEI is a non-rigid polymer and as such this may allow PEI to 'mould' itself into the surface of the silane and not provide any assistance to the problem of surface steric hindrances. PEI has no set conformation and may change this upon variances in the coating temperature. Also, it is not certain as to how many free amine groups are available for reaction and it may be that PEI does not produce any increased surface functionality.

Secondly, it is not until the amino modified-dextran 40k and the different generations of dendrimer are used that any significant signal enhancement is seen. Dextran contains a non-flexible backbone made up of repeating cyclic sugar units and as such has no way of moulding into the surface of the silane, rather it provides a rigid barrier for further activation. Dextran is thus able to enhance surface uniformity and lend itself to the removal of steric hindrances for oligonucleotide hybridisation, allowing a means of signal enhancement that PEI could not effect.

Finally, there is a direct correlation between the size of dendrimer used and its signal enhancing properties. The generation 3 dendrimer has 32 amine groups available on the branches of the dendrimer whereas the higher generation 4 has 64 amine groups available for activation. The table shows that signals increase from ≈ 40000 - 45000 in the case of the generation 3 dendrimer to ≈ 55000 - 60000 in the case of the generation 4 dendrimer. This result shows the importance of increased surface functionality as the dendrimers are expected to have the same basic shape and conformation, the only difference being the increased size and functionality of the generation 4 dendrimer. Also, the signals may be enhanced with the use of dendrimers as they impart a three-dimensional quality onto the surface. The dendrimers are three-dimensional spheres and as such this increased dimensionality may help to improve the signal intensities observed.

A surprising positive aspect that arises from the use of 1,4-phenylenediisothiocyanate (PDITC) as a bifunctional, activating linker. For the purpose of this investigation surfaces have been produced that not only have been terminated with the isothiocyanate containing PDITC, but also other surfaces that are terminated with epoxy and succinimidyl functionalities.

There are three main factors that contribute to spot quality - the hydrophobicity of the surface, the functionality present on the surface and finally the homogeneity of the surface coating. The structure of the bifunctional activating agent can influence all three of these factors and is important in producing consistent, reproducible micro-arrays, necessary for modern diagnostic applications.

### (1) Hydrophobicity of the surface.

This aspect is linked to the carbon content of the bifunctional linker and can be regarded as the repulsion between the solid surface and the aqueous spotting solution. In practice, it is measured by contact angle studies and as a general rule of thumb, the more hydrophobic the surface the more prominent the spot, lending itself to a high contact angle value. Contact angle measurements on surfaces terminated with PDITC have shown themselves to be very hydrophobic in nature, with results never dropping below 70°. This is due to the aromatic benzene-type spacer found in PDITC. With unmodified benzene, the six π-electrons are delocalised throughout the benzene ring and no permanent dipole exists for PDITC to have a strong affinity for water. With the bifunctionalised PDITC, the π-electrons are distorted to a degree but not enough for the linker to produce a hydrophilic surface. As such, use of PDITC in arrays produces a surface where spots do not spread but rather stand prominent. The prominence of the spot means that it will dry in a slightly longer time as its surface area is smaller than a spot that spreads on the surface. This allows a greater time for the probe that is spotted to covalently attach itself to the surface.

### (2) Functionality on the surface.

It is not only a high contact angle that lends itself to high spot quality, but also the nature of the functionality of the activating agent that has a role to play in producing reproducible and effective microarrays. In the context of oligonucleotide immobilisation, the attachment of oligos modified with a hexylamine moiety to an activated surface is desirable. This means that the nature of the activating agent must react quickly and irreversibly with an amine. For this purpose there are a host of bifunctional activating agents with a variety of amine-coupling chemistries that can be used. The rate of this reaction is obviously controlled by the nature of the functionality present at the termini of the bifunctional linker. It is proposed that due to the fast kinetics that occur between amines and isothiocyanate functionalities, surfaces activated with PDITC are more amenable to producing symmetrical and reproducible spots. This can be shown from analysis of the spot profiles that are produced from each spot of the array. A spot profile is a plot of relative signal intensity against the dimension of the spot and shows the spread of functionalities across the area of the spot. Surface functionalities that react quickly and irreversibly produce spot profiles that have a normal distribution profile. However, surface functionalities that react kinetically slowly produce 'dough-nutting' and have 'M' shaped spot profiles. 'Doughnuts' are spots that are seen to produce regions in the centre of the spot that are not as intense as the outer ring of the spot. These have a negative effect on both the quality and reproducibility of the microarray.

A second factor linked to the functionality of the bifunctional agent concerns the nature of the spacing portion of the linker. As discussed in the previous section, PDITC possesses a rigid benzene ring as the bridge between the two isothiocyanate arms. This means that PDITC cannot 'bend-over' after initial attachment of the first isothiocyanate arm, allowing the second arm to then also react with the surface, producing an unactivated surface. This is not the case with linkers that are bridged with flexible alkyl spacers. In many cases it is thermodynamically favourable for these types of linkers to form a pseudo-intermolecular ring. Obviously this limits the amount of functionalities present on the surface and may help to contribute to the poorer performance of these linkers when compared to PDITC.

### (3) Homogeneity of the surface.

This final aspect of producing arrays with a superior spot quality may be directly linked to the bifunctional agent, but has more to do with the surface chemistry preceding activation. However, the nature of the activating arms of the linker does play a part in producing an even, homogeneous surface coating. For this purpose, a linker is required to have its terminal groups capable of reacting quickly and irreversibly within the time frame of the activating procedure. PDITC, possessing isothiocyanate termini, falls into this category as these functionalities react in this manner with any amino groups present on the surface.

The present invention recognises that good spot quality is essential for the production of reliable and reproducible microarrays. One way of qualitatively comparing different surfaces to determine spot quality, is by visual inspection both of the images produced and their subsequent spot profiles. From such an analysis it is observed that those surfaces activated with PDITC produce arrays with no discernable dough-nutting and spot profiles that resemble the desired standard deviation curve. The surfaces terminated with other functionalities can be seen to have both dough-nutting and 'M' shaped spot profiles.

## Claims

1. A process for the manufacture of a solid support comprising a linker molecule capable of attaching a biomolecule to the solid support, comprising:
attaching to the surface of a solid support, a silane with an electrophilic terminal group;
contacting the terminal group with a polyamine; and
activating the polyamine with an electrophilic, bifunctional linker.

2. A process according to claim 1, wherein the silane is an alkoxy silane.

3. A process according to claim 1 or claim 2, wherein the silane is GOPTS or GOPS MDM.

4. A process according to any preceding claim, wherein the polyamine is an amine-terminated non-linear or dendrimeric molecule.

5. A process according to any of claims 1 to 3, wherein the polyamine is amino-modified dextran.

6. A process according to any preceding claim, wherein the electrophilic, bifunctional linker has the general formula:
X-R-X
wherein R is an alkyl, aryl or heteroaryl group, and X is selected from the group consisting of isothiocyanate, isocyanate, epoxide, carbonylimidazole and N-hydroxysuccinimide.

7. A process according to any preceding claim, wherein the electrophilic, bifunctional linker is PDITC.

8. A process according to any preceding claim, wherein the solid support is a ceramic material.

9. A process according to any preceding claim, further comprising the attachment of an amino-capped biomolecule.

10. A process according to claim 9, wherein the biomolecule is a polynucleotide or a protein.

11. A functionalised solid support material obtainable by a process according to any preceding claim.

12. Use of a solid support material according to claim 11, in a diagnostic assay procedure.

13. Use according to claim 12, wherein the assay is a DNA hybridisation assay.
